(19) Europäisches Patentamt European Patent Office Office européen des brevets

(11) **EP 2 990 012 B1**

(12) **EUROPEAN PATENT SPECIFICATION**

(45) Date of publication and mention
of the grant of the patent:
**06.11.2019 Bulletin 2019/45**

(21) Application number: **13883045.0**

(22) Date of filing: **18.09.2013**

(51) Int Cl.:
**A61F 2/16** (2006.01)

(86) International application number:
**PCT/CN2013/001106**

(87) International publication number:
**WO 2014/172816 (30.10.2014 Gazette 2014/44)**

(54) **ASPHERICAL INTRAOCULAR LENS**

ASPHÄRISCHE INTRAOKULARLINSE

LENTILLE INTRAOCULAIRE ASPHÉRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.04.2013 CN 201320204627 U**

(43) Date of publication of application:
**02.03.2016 Bulletin 2016/09**

(73) Proprietor: **Eyebright Medical Technology
(Beijing) Co., Ltd.
Beijing 102200 (CN)**

(72) Inventor: **WANG, Zhao
Beijing 102200 (CN)**

(74) Representative: **Berkenbrink, Kai-Oliver
Patentanwälte Becker & Müller
Turmstrasse 22
40878 Ratingen (DE)**

(56) References cited:
**WO-A1-2009/029515        WO-A1-2011/153158
CN-A- 102 247 222          CN-U- 202 446 298
CN-Y- 201 404 311          US-A1- 2009 204 211
US-A1- 2012 262 670**

**Description**

Field of the Invention

The present invention mainly relates to a new-type aspherical intraocular lens. Specifically, the present invention relates to an aspherical intraocular lens with a spherical aberration ranging from -0.28 $\mu$m to -0.55 $\mu$m.

Background of the Invention

An intraocular lens (IOL) is an artificial lens which is used for implantation into a human eye to replace a natural lens in the human eye which becomes opacified due to the cataract disease, or which is used in a refractive operation to correct vision of the human eye. The flexible foldable intraocular lenses are generally classified into one-piece type and three-piece type according to the engagement manner of the effective optic area and the haptics. In the one-piece type flexible foldable intraocular lens, the effective optic area and the support haptics are integral and made of the same piece of soft material. In the three-piece type flexible foldable intraocular lens, the effective optic area and the support haptics are processed separately and then combined and connected together. Document CN-U-20244628 discloses an aspherical intraocular lens according to the preamble of claim 1.

The intraocular lens maintains at a relative position in a capsule bag in the human eye by means of the interaction force between the support hapics and the capsule bag after being implanted into the human eye. In this note, when light enters from a substance into another substance with different optical density, propagation direction of the light will deflect. This phenomenon is called refraction phenomenon. Diopter indicates a magnitude of the refraction phenomenon (refractive power), with a measure unit of diopter ("D" in short). 1D refractive power is equivalent to focusing parallel light rays on a 1-meter focal length. The action of the eye refracting light rays is called refraction. Focal power of light is used to represent capability of refraction, and is also called diopter. The diopter is the lens's refraction intensity for the light rays. The diopter is a measure unit of refractive power and represented as D. When parallel light rays pass through the refraction substance, and the refractive power of the refraction substance at the 1-meter focusing point is 1 diopter or 1D. As for a lens, the diopter refers to the measure unit of a focal power of the lens, e.g., when a focal length of a lens is 1M, the refractive power of the lens is 1D diopter, inversely proportional to the focal length. The refractive power of the lens is F=1/f, wherein f is the focal length of the lens. In the equation, the measure unit of the refractive power is diopter with a symbol of D, a dimension of $L^{-1}$, $1D=1m^{-1}$.

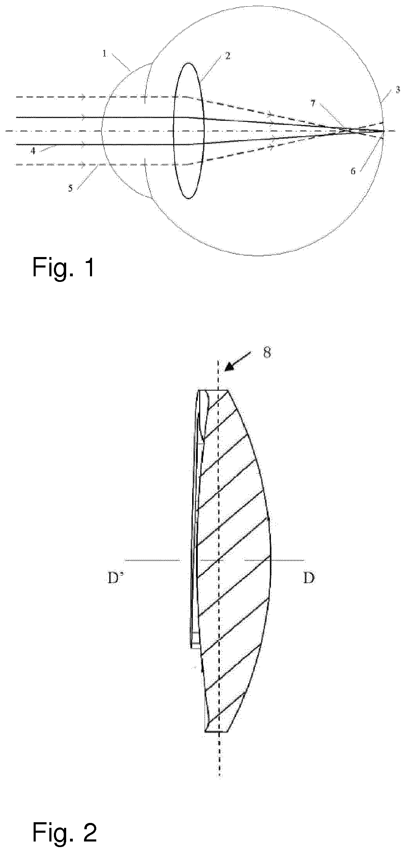

Fig.1 is a schematic view of refraction of light rays in an IOL eye. Cornea 1 and IOL 2 in the IOL eye are both lenses. The cornea is a lens of an aspherical degree, and bears over 70% of the refracting power of the human eye. The IOL in replacement of the natural lens in the eye of a cataract sufferer bears about 30% of the refracting power of the eye. Though the cornea has an aspherical degree, it is insufficient to correct the spherical aberration of the cornea itself. A huge amount of clinical statistics shows that the cornea of a human eye has a positive spherical aberration. When the pupil of the human eye is relatively small (in a bright environment), light rays enter into the eye in thin beams; the spherical aberration is small; and the image points are converged substantially on retina 3. When the pupil is relatively large (in a dark environment), light rays enter into the eye with a large aperture; edge light rays 5 are converged on focal point 7; and paraxial rays 4 are converged on ideal image point 6, whereby the edge light rays 5 and paraxial rays 4 are not converged on the same point (as shown in Fig.1), and the human eye has a large spherical aberration which leads to blurred vision.

In an IOL eye from which the natural lens is removed, the spherical aberration is a sum of the spherical aberration of the cornea and the spherical aberration of the IOL. The conventional spherical IOL has a positive spherical aberration, increasing the total spherical aberration of the human eye on the basis of the positive spherical aberration of the cornea and thus lowering the visual quality after the surgery. In order for the patient to have a better vision, the total spherical aberration of the eye after the surgery needs to be reduced. Based on this theory, the aspherical IOL has been developed. Thanks to its aspherical surface design, the aspherical IOL has zero or a negative spherical aberration, so as to achieve a low level of total spherical aberration of the eye after the surgery, thereby solving the problem of reduced contrast caused by the ordinary spherical IOL and enhancing the visual quality in dark and night environment.

In 1991, Atchison developed an aspherical IOL with a quadric surface, which achieves proper aberrations and change of the refraction state. However, the optimum optical performance of this aspherical IOL depends greatly on the position and the extent of off-centering of the lens. In 1999, Wenner et al. reduced aberrations with one side of the IOL aspherical surface and changed the radius of the other side to obtain a greater refractive power. Results showed that an aspherical IOL could improve the visual quality.

A great progress has been made in the development of the aspherical IOL in the twentieth century, thanks to the application of wave-front aberration technology in IOL design. The Tecnis Z900 type IOL manufactured by AMO is the first type of IOL to use the wave-front aberration technology to avoid aberrations, and is the first type of IOL that can generate a negative spherical aberration. It can generate a negative spherical aberration of -0.27$\mu$m. The value of the spherical aberration of this type of aspherical IOL is selected to be equal to the statistical average spherical aberration of the cornea. The design concept is to completely offset the positive spherical aberration of the cornea. More companies have subsequently developed aspherical IOLs of various types. The aspherical IOL Acrysof SN60WF, also called IQ, designed later by Alcon adopts an aspherical design for the effective optical area posterior surface which can generate a negative spherical aberration of -0.2μm, so that the entire eye has a positive spherical aberration so as to improve the visual comfort. The SofPort AO and Akreos AO aspherical IOLs made by Bausch&Lomb have no aberrations, so as to reduce the impact of lens off-centering and tilting on the image quality. Alcon (owned by Novartis now), AMO (owned by Abbott now) and Bausch&Lomb of the United States are the world's major producers of IOL.

Aspherical design is now widely used in IOLs. The function of the aspherical surface is to compensate the positive spherical aberration generated by the cornea such that the total spherical aberration of an IOL eye is at a relatively low level. The shape and spherical aberration of the cornea play a key role in the design of the aspherical surface. The prior-art aspherical IOLs are all designed on the basis of statistics on corneas of westerners. They generally adopt such universal human eye models as Liou and Navarro. The cornea spherical aberration is set at the average statistical value +0.27μm, and the spherical aberration correction amount of the IOL is controlled within -0.27μm.

However, it was pointed out in a paper issued by Kooi Ling Lim, a Hong Kong physician, that the cornea Q value of people in southern China and Southeast Asia is slightly smaller than that of the Europeans, and the cornea spherical aberration of the former is slightly larger than that of the latter (Kooi Ling Lim, Boptom (Hons), Han Bor Fam, FRCSE. Ethnic differences in higher-order aberrations: Spherical aberration in the South East Asian Chinese eye. J Cataract refractive surg. 2009;35:2144-2148). Additionally, statistics show that by examining Chinese cataract patients with aspherical IOL implants, Chinese ophthalmologists have found that over 50% of these cataract patients still have a large residual spherical aberration after implantation with the aspheric IOLs on the market (Yanwen Fang, Yi Lu, Xinhua Wu, Aizhu Miao, Yi Luo. Visual function and subjective quality of life in Chinese cataract patients after implantation with aspheric intraocular lenses. Eur J Ophthalmol. 2011;21(6):732-740). This means that the cornea spherical aberration is not well corrected. Hence, the existing aspherical IOLs on the market cannot well satisfy the needs of cataract patients in China and some other Asian regions.

It can thus be seen that cataract patients in China and some other Asian regions need a new-type aspherical IOL capable of correcting a larger cornea spherical aberration so as to provide better visual quality for the respective cataract patients.

Summary of the Invention

The present invention is proposed in view of the above technical problems. A goal of the present invention is to provide a new-type aspherical IOL. Compared with the aspherical IOLs on the market, the new-type aspherical IOL of the present invention is capable of correcting a larger cornea spherical aberration so that the cataract patient enjoys better visual quality.

Definitions of terms

The term "effective optical area" used in the present application refers to the part which is located at the center of the IOL, has optical properties and thereby can achieve a major function of adjusting IOL diopter.

The term "effective optical area posterior surface" used in the present application refers to the effective optical area surface in contact with the human eye posterior capsule after implantation of the IOL into the human eye.

The term "effective optical area anterior surface" used in the present application refers to the effective optical area surface farther away from the posterior capsule opposite to the effective optical area posterior surface after implantation of the IOL into the human eye.

The term "haptics" or "support haptics" used in the present application refers to a portion which is connected to the IOL effective optical area and functions to support the effective optical area and transfer the retraction force generated by the retraction and varicosity of the ciliary muscle to the effective optical area.

Positional terms "anterior" and "posterior" indicative of orientation relationship used in the present application are relative to the distance away from the human eye posterior capsule. For example, as far as the aspherical intraocular lens in the present application is concerned, "effective optical area posterior surface" refers to an optical surface closer to the human eye posterior capsule than "effective optical area anterior surface".

The terms "convex" and "concave" indicative of shape used in the present application are relative to a longitudinal central plane (designated by reference sign 8 as shown in Fig.2) of the IOL effective optical area.

The term "basic spherical surface" used in the present application refers to a spherical surface corresponding to various surface shapes utilized by the IOL effective optical area anterior and posterior surface of in the present invention. In the present application, to make the terms consistent, the spherical surface is collectively called "basic spherical surface".

The term "effective optical area surface apex" used in the present application refers to a center point of the IOL convex effective optical area anterior surface or the IOL convex effective optical area posterior surface. That is to say, the 'effective optical area surface apex" refers to a center point of the IOL convex effective optical area anterior surface farthest away from the longitudinal central plane of the IOL effective optical area; or a point of the IOL convex effective optical area posterior surface farthest away from the longitudinal central plane of the IOL effective optical area.

Compared with the aspherical IOLs existing on the market, the aspherical IOL of the present invention is capable of correcting a larger cornea spherical aberration so that the cataract patient enjoys better visual quality.

Brief Description of Drawings

The features and advantages of the present invention will be made more apparent according to the following figures and description, wherein:

Fig.1 schematically illustrates a basic composition of a human eye refractive system;
Fig.2 illustrates a cross-sectional view of an intraocular lens according to an embodiment of the present invention, wherein haptics are already folded onto the anterior surface of the effective optical area of the intraocular lens;
Fig.3 schematically illustrates the difference between an effective optical area surface of an aspherical intraocular lens according to an embodiment of the present invention and a corresponding spherical surface;
Fig.4 is a modulation transfer function (MTF) graph of 15.0D IOLs of the present invention with spherical aberrations of -0.28$\mu$m, -0.29$\mu$m, -0.42$\mu$m, and -0.44$\mu$m respectively as actually measured in a human eye model with a cornea spherical aberration of +0.29$\mu$m with a large aperture (5.0 mm) when the pupil diameter is 5.0 mm;
Fig.5 is a modulation transfer function (MTF) graph of 15.0D IOLs of the present invention with spherical aberrations of -0.28$\mu$m, -0.29$\mu$m, -0.42$\mu$m, and -0.44$\mu$m respectively as actually measured in a human eye model with a cornea spherical aberration of +0.42 $\mu$m with a pupil diameter of 5.0 mm; and
Fig.6 is a modulation transfer function (MTF) graph of 15.0D IOLs of the prior art technology with spherical aberrations of -0.20$\mu$m, and -0.27$\mu$m respectively, and of a 15.0D IOL of the present invention with a spherical aberration of -0.29$\mu$m as actually measured in a human eye model with a cornea spherical aberration of +0.29$\mu$m with a pupil diameter of 5.0 mm.

The same reference signs used in the figures of the present application denote identical or similar elements.

Listing of parts denoted by reference signs

1 cornea
2 intraocular lens (IOL)
3 retina
4 paraxial light rays
5 edge light rays
6 ideal image point
7 edge light rays focal point
8 longitudinal central plane of an effective optical area of the intraocular lens
D-D' ocular axis direction

Detailed Description of Preferred Embodiments

The materials for preparing the intraocular lens of the present invention are generally classified into silicone, hydrophilic acrylate (hydrogel), hydrophobic acrylate, and polymethyl methacrylate (PMMA). The refractive indices of examples of the following materials adopted by the IOL of the present invention all are between 1.45 and 1.56 under the condition of 25°C. As known by those skilled in the art, a conventional preparation method may be adopted according to needs to enable the prepared materials to have any refractive index between 1.45 and 1.56.

The IOL effective optical area anterior surface and posterior surface of the present invention can be flat-convex, convex-flat or equal double-convex with respect to the longitudinal central plane of the IOL effective optical area.

The surface shape of the IOL effective optical area anterior surface or posterior surface of the present invention may comprise a spherical surface, an aspherical surface and a flat surface. An aspherical surface design is intended to further improve the imaging quality of the basic spherical surface of the IOL. The IOL effective optical area anterior surface and/or posterior surface of the present invention adopt(s) an aspherical design. Specifically, the surface shape of the aspherical IOL effective optical area anterior surface, the surface shape of the aspherical IOL effective optical area posterior surface, or the surface shapes of the aspherical IOL effective optical area anterior surface and posterior surface of the present invention can be aspherical.

To configure the effective optical area surface of the present invention as aspherical is equivalent to adding an aspherical design on the basic spherical surface of the IOL effective optical area.

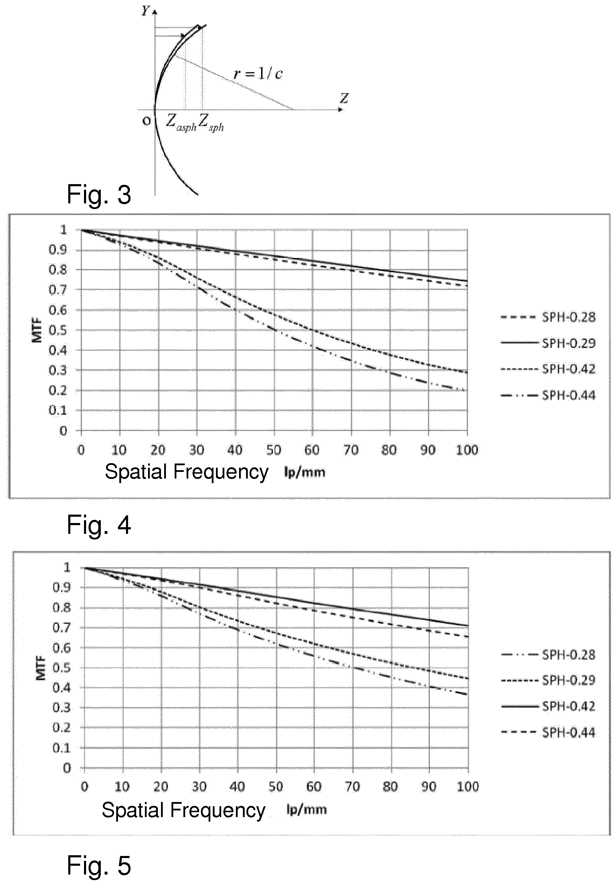

To more accurately describe the surface shape of the effective optical area of the IOL of the present invention, a two-dimensional coordinate system is established with an effective optical area surface apex adopting an aspherical surface design in the IOL of the present invention as an original point as shown in Fig.3, a longitudinal coordinate axis Y of the coordinate system is tangential with the effective optical area surface and passes through the effective optical area surface apex O; a horizontal coordinate axis Z of the coordinate system is parallel to an ocular axis direction D-D' and is at an angle of 90 degrees relative to the longitudinal coordinate axis Y and passes through the effective optical area surface apex O as shown in Fig.2. Points on the effective optical area surface adopting the aspherical surface design in the IOL of the present invention are in rotational symmetrical relationship with the horizontal coordinate axis Z which passes through the effective optical area surface apex O and is parallel to an ocular axis direction D-D' as shown in Fig.2. Therefore, the surface shape of the effective optical area surface adopting the aspherical surface design in the IOL of the present invention can be restored through rotational symmetrical conversion on the premise of defining the coordination relationship of the effective optical area surface adopting the aspherical surface design in the IOL of the present invention on a plane constituted by the longitudinal coordinate axis Y and the horizontal coordinate axis Z. Respective points on the effective optical area surface adopting the aspherical surface design in the IOL of the present invention on the plane constituted by the longitudinal coordinate axis Y and the horizontal coordinate axis Z may be represented as (Z, y). As shown in Fig.3, $Z_{asph}$ is a Z value of any point of a curve of the aspherical surface shape on the two-dimensional coordination system plane YZ, and $Z_{sph}$ is a Z value Z of a curve of the spherical surface shape on the two-dimensional coordination system plane YZ.

A curve of the aspherical surface of the obviously rearwardly convex IOL effective optical area surface of the present invention on the two-dimensional coordinate system plane YZ satisfies the following expression of aspherical surface design expression:

$$Z(y) = \frac{cy^2}{1+\sqrt{1-c^2 y^2}} + \sum_{i=m}^{n} A_{2i} \bullet y^{2i} \qquad (1)$$

wherein Z(y) is an expression of the curve of the aspherical surface of the IOL effective optical area on the YZ plane, c is a reciprocal of a radius of curvature of the surface of the basic spherical surface of the effective optical area, y is a vertical distance of any point on the curve from the horizontal coordinate axis Z, $A_{2i}$ is a higher-order term coefficient of the aspherical surface, m, n are both an integer greater than or equal to 1 and n≥m, these terms reflect the magnitude of difference between the aspherical surface shape and the basic spherical surface shape. It can be seen from the above equation that the aspherical surface can be considered as a superposition of the basic spherical surface term $\frac{cy^2}{1+\sqrt{1-c^2 y^2}}$ and a deviation quantity, wherein the aspherical surface higher order coefficient $\sum_{i=m}^{n} A_{2i} \bullet y^{2i}$ is

a superposed term.

Points on the aspherical surface shape of the IOL of the present invention are obtained in a way that the curve rotates about the horizontal coordinate axis (Z) for symmetry variation.

Points on the aspherical surface of the IOL of the present invention satisfy the above-mentioned aspherical surface design expression (1), and parameter values m=2 and n=5 of superposed terms in the aspherical surface design expression of the IOL of the present invention.

A radius of curvature of the basic spherical surface of the effective optical area posterior surface of the aspherical IOL of the present invention is preferably smaller than that of a basic spherical surface of the effective optical area anterior surface of the aspherical IOL of the present invention, so as to not only improve stability of a position of the aspherical IOL of the present invention in a capsule bag, facilitate reduction of an incidence rate of posterior capsule opacification (PCO) after implantation of the IOL, but also correct a larger cornea spherical aberration.

Moreover, a thickness at a center (CT) of the effective optical area of the IOL of the present invention is in a range of 0.6 mm-1.3 mm. "A thickness at a center of the effective optical area" refers to a thickness at the thickest position at the center of the effective optical area of the IOL of the present invention. As publicly known by those skilled in the art, the magnitude of the thickness at a center of the effective optical area of the IOL of the present invention depends on the utilized material and the achieved diopter. These IOLs of the present invention all can achieve a diopter of 5.0D-36.0D. The spherical aberration of the aspherical IOL of the present invention ranges between -0.28μm and -0.55μm. The aspherical IOL of the present invention can be a one-piece IOL or a three-piece IOL.

In this art, MTF graph is an effective, objective and full image quality evaluating means. In the practical sense, a MTF value represents contrast and sharpness of an optical image, is measured by how many lines are presented in a one-millimeter scope, and has a measure unit lp/mm.

Fig.4 is a modulation transfer function (MTF) graph of 15.0D IOLs of the present invention with spherical aberrations of -0.28μm, -0.29μm, -0.42μm, and -0.44μm respectively as actually measured in a human eye model with a cornea spherical aberration of +0.29 μm with a larger aperture (5.0 mm) when the pupil diameter is 5.0 mm. As shown in the figure, as far as a human eye model with a cornea spherical aberration of +0.29μm is concerned, 15.0D IOLs of the present invention with spherical aberrations of -0.28μm and -0.29μm respectively can better correct the cornea spherical aberration, compared with 15.0D IOLs of the present invention with spherical aberrations of -0.42μm and -0.44μm respectively. The IOL of the present invention provides the possibility of nicely compensating a lager human eye cornea spherical aberration, compared with the prior-art aspherical IOL. The IOL of the present invention can achieve a spherical aberration of the entire eye of infinitely close to zero, i.e., the negative spherical aberration value of the IOL is equivalent to the positive spherical aberration value of the cornea.

Fig.5 is a modulation transfer function (MTF) graph of 15.0D IOLs of the present invention with spherical aberrations of -0.28μm, -0.29μm, -0.42μm, and -0.44μm respectively as actually measured in a human eye model with a cornea spherical aberration of +0.42μm with a pupil diameter of 5.0 mm. As shown in the figure, as far as a human eye model with a cornea spherical aberration of +0.42μm is concerned, 15.0D IOLs of the present invention with spherical aberrations of -0.42μm and -0.44μm respectively correct the cornea spherical aberration better than 15.0D IOLs of the present invention with spherical aberrations of -0.28μm and -0.29μm respectively. The IOL of the present invention provides the possibility of nicely compensating a lager human eye cornea spherical aberration, compared with the prior-art aspherical IOL. The IOL of the present invention can achieve a spherical aberration of the entire eye of infinitely close to zero, i.e., the negative spherical aberration value of the IOL is equivalent to the positive spherical aberration value of the cornea.

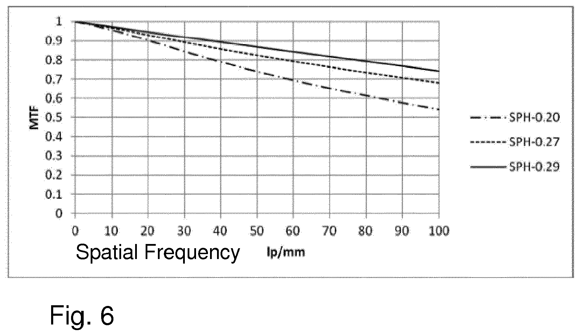

Fig.6 is a modulation transfer function (MTF) graph of 15.0D IOLs of the prior art technology with spherical aberrations of -0.20μm and -0.27μm respectively, and of a 15.0D IOL of the present invention with a spherical aberration of -0.29μm as actually measured in a human eye model with a cornea spherical aberration of +0.29μm with a pupil diameter of 5.0 mm. As shown in the figure, as far as a human eye model with a cornea spherical aberration of +0.29μm is concerned, a 15.0D IOL of the present invention with a spherical aberration of -0.29μm corrects the cornea spherical aberration better than prior-art 15.0D IOLs with spherical aberrations of -0.20μm and -0.27μm respectively. The IOL of the present invention can achieve a spherical aberration of the entire eye of infinitely close to zero, i.e., the negative spherical aberration value of the IOL is equivalent to the positive spherical aberration value of the cornea. Compared with the prior-art aspherical IOL, the IOL of the present invention is advantageous in compensating a greater human eye cornea spherical aberration so that the cataract patient enjoys better visual quality.

As seen from the above figures, the new-type aspherical IOL of the present invention can correct a greater cornea spherical aberration so that the cataract patient enjoys better visual quality.

Embodiments

The following Tables 1-11 list some surface shape design embodiments of the IOL of the present invention, wherein Ra is a radius of curvature of the basic spherical surface of the IOL anterior surface (in millimeter) of the present invention; Rp is a radius of curvature of the basic spherical surface of the IOL posterior surface (in millimeter) of the present invention; the surface is outwardly convex relative to a longitudinal central plane of the effective optical area of the IOL if the value of the radius of curvature is a positive number; $C_T$ is a center thickness of the effective optical area of the IOL of the present invention; D represents the diopeter of the IOL of the present invention; and A4, A6, A8, A10 are high-order term coefficient values of the aspherical surface of the IOL (In expression 1, m=2, and n=5. In the same optical surface shape structure, the amount of the spherical aberration is mostly related with A4. A6, A8 and A10 are also related with the spherical aberration, but are mainly for compensating other high-order aberrations such as comatic aberration).

Table 1 A maximum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -15 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.36 | Infinite | 25.233 | 0.603 | -9.008957 E-04 | -5.473346 E-05 | 8.855133 E-06 | -1.445474 E-07 |
| 5.0 | -0.35 | 51.568 | 51.568 | 0.603 | -8.672406 E-04 | -5.732971 E-05 | 8.915716 E-06 | -1.355270 E-07 |
| 5.0 | -0.35 | 26.366 | Infinite | 0.603 | -8.451413 E-04 | -5.936512 E-05 | 8.864405 E-06 | -1.209806 E-07 |
| 36.0 | -0.28 | 5.206 | 15.0 | 1.234 | -1.409406 E-03 | -6.624055 E-05 | 2.503216 E-06 | 2.985309 E-07 |
| 36.0 | -0.52 | 7.487 | 7.487 | 1.234 | -1.801696 E-03 | -1.017574 E-04 | 1.013746 E-05 | -5.733992 E-08 |
| 36.0 | -0.55 | 13.638 | 5.0 | 1.234 | -2.899540 E-03 | -2.368207 E-04 | 4.227739 E-05 | -1.866745 E-06 |

Table 2 A minimum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.28 | Infinite | 25.368 | 0.603 | -7.262697 E-04 | -3.737443 E-05 | 6.132308 E-06 | -7.655982 E-08 |
| 5.0 | -0.28 | 51.824 | 51.824 | 0.603 | -7.046294 E-04 | -4.102339 E-05 | 6.462907 E-06 | -8.005722 E-08 |
| 5.0 | -0.28 | 26.482 | Infinite | 0.603 | -7.000851 E-04 | -4.504268 E-06 | 6.860163 E-06 | -8.485116 E-08 |
| 36.0 | -0.28 | 5.206 | 15.0 | 1.234 | -1.409406 E-03 | -6.624055 E-05 | 2.503216 E-06 | 2.985309 E-07 |
| 36.0 | -0.28 | 7.512 | 7.512 | 1.234 | -1.271187 E-03 | -1.865361 E-05 | -3.411270 E-06 | 3.971910 E-07 |
| 36.0 | -0.28 | 13.936 | 5.0 | 1.234 | -1.444860 E-03 | -6.263703 E-05 | -4.049773 E-05 | 2.744572 E-06 |

Table 3 A maximum negative spherical aberration design of 5D and 36D (in consideration that other high-order aberrations, mainly comatic aberration and trefoil aberration besides spherical aberration are added during aberration optimization of the lens). The 5D surface shapes are respectively flat-convex, convex-flat, and equal double-convex. Since 36D is unsuitable for extreme surface shape design, the radii of curvature of the posterior surface are set as -15 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration μm | Ra mm | Rp mm | $C_T$ mm | A4 | A6 | A8 | A10 |
|---|---|---|---|---|---|---|---|---|
| 5.0 | -0.35 | 56.313 | 45.5 | 0.603 | -8.719199 E-04 | -7.827711 E-05 | 1.340746 E-05 | -3.717450 E-07 |
| 8.5 | -0.36 | 23.533 | 45.5 | 0.652 | -8.801546 E-04 | -8.135024 E-05 | 1.337447 E-05 | -3.487032 E-07 |
| 9.0 | -0.38 | 55.195 | 19.5 | 0.653 | -9.584368 E-04 | -7.771218 E-05 | 1.313784 E-05 | -3.463388 E-07 |
| 14.5 | -0.39 | 17.514 | 19.5 | 0.758 | -9.773904 E-04 | -8.395224 E-05 | 1.328628 E-05 | -3.192171 E-07 |
| 15.0 | -0.44 | 41.562 | 11.1 | 0.771 | -1.187400 E-03 | -7.933591 E-05 | 1.279587 E-05 | -3.025497 E-07 |
| 36.0 | -0.38 | 5.797 | 11.1 | 1.234 | -1.474191 E-03 | -1.205246 E-04 | 1.367587 E-05 | -2.402575 E-07 |

Table 4 A minimum negative spherical aberration design of 5D and 36D (in consideration that other high-order aberrations, mainly comatic aberration and trefoil aberration besides spherical aberration are added during aberration optimization of the lens). The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration μm | Ra mm | Rp mm | $C_T$ mm | A4 | A6 | A8 | A10 |
|---|---|---|---|---|---|---|---|---|
| 5.0 | -0.28 | 56.864 | 45.5 | 0.603 | -6.978255 E-04 | -5.666711 E-05 | 9.500535 E-06 | -2.350676 E-07 |
| 8.5 | -0.28 | 23.646 | 45.5 | 0.652 | -6.915847 E-04 | -5.764356 E-05 | 9.257616 E-06 | -2.115973 E-07 |
| 9.0 | -0.28 | 55.998 | 19.5 | 0.653 | -7.146027 E-04 | -4.925470 E-05 | 8.134350 E-06 | -1.819366 E-07 |
| 14.5 | -0.28 | 17.606 | 19.5 | 0.758 | -7.161689 E-04 | -5.151218 E-05 | 7.855059 E-06 | -1.488852 E-07 |
| 15.0 | -0.28 | 42.343 | 11.1 | 0.771 | -8.025291 E-04 | -3.568517 E-05 | 5.266991 E-06 | -6.565346 E-08 |
| 36.0 | -0.28 | 5.808 | 11.1 | 1.234 | -1.262051 E-03 | -8.571288 E-05 | 8.356508 E-06 | -7.795887 E-08 |

Table 5 A maximum negative spherical aberration design of 5D and 36D (in consideration that other high-order aberrations besides spherical aberration are added during aberration optimization of the lens, and in consideration of the rotation and off-centering confrontation performance of the lens). The 5D surface shapes are respectively flat-convex, convex-flat, and equal double-convex. Since 36D is unsuitable for extreme surface shape design, the radii of curvature of the posterior surface are set as -15 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.32 | 56.804 | 45.5 | 0.603 | -8.71397 E-04 | -9.777972 E-05 | 2.031675 E-05 | -8.073296 E-07 |
| 8.5 | -0.35 | 24.115 | 45.5 | 0.652 | -8.794724 E-04 | -5.714701 E-05 | 8.789938 E-06 | -1.232855 E-07 |
| 9.0 | -0.38 | 56.661 | 19.5 | 0.653 | -9.577149 E-04 | -5.252387 E-05 | 8.541138 E-06 | -1.270855 E-07 |
| 14.5 | -0.38 | 17.933 | 19.5 | 0.758 | -9.765776 E-04 | -5.354654 E-05 | 8.097157 E-06 | -8.373005 E-08 |
| 15.0 | -0.42 | 42.471 | 11.1 | 0.771 | -1.186463 E-03 | -5.674722 E-05 | 9.237949 E-06 | -1.501547 E-07 |
| 36.0 | -0.36 | 5.826 | 11.1 | 1.234 | -1.469441 E-03 | -7.559095 E-05 | 6.027625 E-06 | 1.121759 E-07 |

Table 6 A minimum negative spherical aberration design of 5D and 36D (in consideration that other high-order aberrations besides spherical aberration are added during aberration optimization of the lens, and in consideration of the rotation and off-centering confrontation performance of the lens). The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration $\mu$m | Ra mm | Rp mm | $C_T$ mm | A4 | A6 | A8 | A10 |
|---|---|---|---|---|---|---|---|---|
| 5.0 | 0.28 | 58.019 | 45.5 | 0.603 | -6.971289 E-04 | -3.955366 E-05 | 6.250134 E-06 | -7.461521 E-08 |
| 8.5 | 0.28 | 24.235 | 45.5 | 0.652 | -7.053803 E-04 | -3.645538 E-05 | 5.441448 E-06 | -2.421245 E-08 |
| 9.0 | 0.28 | 57.731 | 19.5 | 0.653 | -7.255387 E-004 | -2.488131 E-05 | 4.149995 E-06 | -2.580623 E-10 |
| 14.5 | 0.28 | 18.056 | 19.5 | 0.758 | -7.587056 E-04 | -1.901607 E-05 | 2.137582 E-06 | 1.330336 E-07 |
| 15.0 | 0.28 | 42.806 | 11.1 | 0.771 | -8.235960 E-04 | -3.333062 E-05 | 5.518782 E-06 | -8.956342 E-08 |
| 36.0 | 0.28 | 5.831 | 11.1 | 1.234 | -1.309008 E-03 | -5.783223 E-05 | 3.845280 E-06 | 1.365105 E-07 |

Table 7 A negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.48.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.29 | 60.606 | 45.5 | 0.603 | -7.344029 E-04 | -3.982893 E-05 | 5.940338 E-06 | -3.661740 E-08 |
| 8.5 | -0.29 | 24.238 | 45.5 | 0.652 | -7.344029 E-04 | -3.982893 E-05 | 5.940338 E-06 | -3.661740 E-08 |
| 9.0 | -0.30 | 59.140 | 19.5 | 0.653 | -7.876327 E-04 | -2.065663 E-05 | 2.380351 E-06 | 1.300165 E-07 |
| 14.5 | -0.29 | 18.144 | 19.5 | 0.758 | -7.876327 E-04 | -2.065663 E-05 | 2.380351 E-06 | 1.300165 E-07 |
| 15.0 | -0.29 | 44.964 | 11.1 | 0.771 | -8.566558 E-04 | -3.805036 E-05 | 6.225801 E-06 | -1.103511 E-07 |
| 36.0 | -0.29 | 5.854 | 11.1 | 1.234 | -1.319197 E-03 | -5.915059 E-05 | 4.008839 E-06 | 1.378898 E-07 |

Table 8 A maximum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -15 mm (forwardly convex and rearwardly flat), equal double-convex, and -5 mm (forwardly flat and rearwardly convex) respectively. The material of the IOLs is hydrophobic acrylate of a refractive index of 1.46.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.36 | Infinite | 22.058 | 0.603 | -1.019849 E-03 | -8.481911 E-05 | 1.489944 E-05 | -4.211297 E-07 |
| 5.0 | -0.35 | 45.087 | 45.087 | 0.603 | -9.746084 E-04 | -8.825643 E-05 | 1.503908 E-05 | -4.147512 E-07 |
| 5.0 | -0.35 | 23.056 | Infinite | 0.603 | -9.464353 E-04 | -9.147780 E-05 | 1.514737 E-05 | -4.070746 E-07 |
| 15.0 | -0.48 | Infinite | 7.734 | 0.711 | -1.637259 E-03 | -8.352639 E-05 | 1.311029 E-05 | -2.859604 E-07 |
| 15.0 | -0.40 | 15.922 | 15.922 | 0.711 | -1.131804 E-03 | -9.438117 E-05 | 1.470381 E-05 | -3.412040 E-07 |
| 15.0 | -0.32 | 8.224 | Infinite | 0.711 | -9.874183 E-04 | -1.042993 E-04 | 1.528260 E-05 | -3.521017 E-07 |
| 36.0 | -0.90 | 20.0 | 3.909 | 1.234 | -4.528593 E-03 | -2.837436 E-05 | -3.422158 E-05 | 2.092757 E-06 |
| 36.0 | -0.55 | 6.710 | 6.710 | 1.234 | -2.163577 E-03 | -1.393514 E-04 | 1.302807 E-05 | -1.145586 E-07 |
| 36.0 | -0.33 | 5.0 | 10.968 | 1.234 | -1.746849 E-03 | -1.314449 E-04 | 1.208061 E-05 | -1.274738 E-07 |

Table 9 A minimum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.46.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.28 | Infinite | 22.170 | 0.603 | -8.077360 E-04 | -5.967397 E-05 | 1.027773 E-05 | -2.587878 E-07 |
| 5.0 | -0.28 | 45.301 | 45.301 | 0.603 | -7.792731 E-04 | -6.397575 E-05 | 1.066658 E-05 | -2.624212 E-07 |
| 5.0 | -0.28 | 23.156 | Infinite | 0.603 | -7.675947 E-04 | -6.833274 E-05 | 1.105722 E-05 | -2.659226 E-07 |
| 15.0 | -0.28 | Infinite | 7.779 | 0.711 | -1.074851 E-03 | -2.068180 E-05 | 1.868492 E-06 | 8.696226 E-08 |
| 15.0 | -0.28 | 15.969 | 15.969 | 0.711 | -8.217267 E-04 | -5.547618 E-05 | 8.220108 E-06 | -1.382031 E-07 |
| 15.0 | -0.28 | 8.232 | Infinite | 0.711 | -8.896673 E-04 | -8.916194 E-05 | 1.277019 E-05 | -2.670175 E-07 |
| 36.0 | -0.28 | 6.739 | 6.739 | 1.234 | -1.499610 E-03 | -2.856401 E-05 | -5.088897 E-06 | 4.998457 E-07 |

Table 10 A maximum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -15 mm (forwardly convex and rearwardly flat), equal double-convex, and -5 mm (forwardly flat and rearwardly convex) respectively. The material of the IOLs is hydrophobic acrylate of a refractive index of 1.55.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.35 | Infinite | 37.663 | 0.603 | -5.773213 E-04 | -4.930434 E-05 | 8.611147 E-06 | -2.418869 E-07 |
| 5.0 | -0.35 | 76.888 | 76.888 | 0.603 | -5.631943 E-04 | -5.047280 E-05 | 8.661792 E-06 | -2.400269 E-07 |
| 5.0 | -0.35 | 39.265 | Infinite | 0.603 | -5.526794 E-04 | -5.158208 E-05 | 8.700751 E-06 | -2.375209 E-07 |
| 15.0 | -0.44 | Infinite | 13.282 | 0.711 | -7.752361 E-04 | -4.752735 E-05 | 7.990829 E-06 | -1.902140 E-07 |
| 15.0 | -0.39 | 27.304 | 27.304 | 0.711 | -6.316097 E-04 | -5.192137 E-05 | 8.439703 E-06 | -2.053946 E-07 |
| 15.0 | -0.35 | 14.077 | Infinite | 0.711 | -7.752361 E-04 | -4.752735 E-05 | 7.990829 E-06 | -1.902140 E-07 |
| 36.0 | -0.50 | 11.505 | 11.505 | 1.234 | -9.621602 E-04 | -6.053624 E-05 | 8.124785 E-06 | -1.292575 E-07 |

Table 11 A minimum negative spherical aberration design of 5D and 36D. The 5D surface shapes are respectively flat-convex, convex-flat, and equal double-convex. Since 36D is unsuitable for extreme surface shape design, the radii of curvature of the posterior surface are set as -20 mm (forwardly convex and rearwardly flat), equal double-convex, and -5 mm (forwardly flat and rearwardly convex) respectively. The material of the IOLs is hydrophobic acrylate of a refractive index of 1.55.

| Diopter D | Spherical aberration $\mu$m | Ra mm | Rp mm | $C_T$ mm | A4 | A6 | A8 | A10 |
|---|---|---|---|---|---|---|---|---|
| 5.0 | -0.28 | Infinite | 37.838 | 0.603 | -4.640847 E-04 | -3.561504 E-05 | 6.104371 E-06 | -1.536629 E-07 |
| 5.0 | -0.28 | 77.248 | 77.248 | 0.603 | -4.500973 E-04 | -3.647722 E-05 | 6.133682 E-06 | -1.517976 E-07 |
| 5.0 | -0.28 | 39.436 | Infinite | 0.603 | -4.490896 E-04 | -3.834341 E-05 | 6.331845 E-06 | -1.548916 E-07 |
| 15.0 | -0.28 | Infinite | 13.337 | 0.711 | -5.284179 E-04 | -2.014675 E-05 | 3.186303 E-06 | -3.619687 E-08 |
| 15.0 | -0.28 | 27.380 | 27.380 | 0.711 | -4.617116 E-04 | -3.138305 E-05 | 4.954159 E-06 | -9.512166 E-08 |
| 15.0 | -0.28 | 14.099 | Infinite | 0.711 | -4.669927 E-04 | -4.272148 E-05 | 6.552829 E-06 | -1.417242 E-07 |
| 36.0 | -0.28 | 11.543 | 11.543 | 1.234 | -6.389072 E-04 | -1.437094 E-05 | 5.293093 E-07 | 1.158933 E-07 |

The following can be known from the surface shape design embodiments of the IOL of the present invention listed in the above tables.

As far as IOLs of the present invention formed of hydrophobic acrylate of a refractive index of 1.48 and having a diopter between 5D and 36D are concerned, in order to compensate a cornea spherical aberration between +0.28$\mu$m and +0.55$\mu$m, the coefficient $A_4$ in the aspherical surface curve expression of the IOLs of the present invention is between -2.89E-03 and -7.00E-04; the coefficient $A_6$ is between -2.36E-04 and -4.50E-06; the coefficient $A_8$ is between -4.04E-05 and -4.23E-05; and the coefficient A10 is between -1.87E-06 and -2.75E-06. Preferably, as far as IOLs of the present invention formed of hydrophobic acrylate of a refractive index of 1.48 and having a diopter between 5D and 36D are concerned, in order to compensate a cornea spherical aberration between +0.28$\mu$m and +0.44$\mu$m (in consideration that other high-order aberrations, mainly comatic aberration and trefoil aberration besides spherical aberration are added during the aberration optimization of the lens), the coefficient $A_4$ in the aspherical surface curve expression of the IOLs of the present invention is between -1.47E-03 and -6.91E-04; the coefficient $A_6$ is between -1.20E-04 and -3.56E-05; the coefficient $A_8$ is between 5.26E-06 and 1.37E-05; and the coefficient $A_{10}$ is between -3.71E-07 and -6.56E-08. More preferably, as far as IOLs of the present invention formed of hydrophobic acrylate of a refractive index of 1.48 and having a diopter between 5D and 36D are concerned, in order to compensate a cornea spherical aberration between +0.28$\mu$m and +0.42$\mu$m (in consideration that other high-order aberrations besides spherical aberration are added during aberration optimization of the lens, and in consideration of the rotation and off-centering confrontation performance of the lens), the coefficient $A_4$ in the aspherical surface curve expression of the IOLs of the present invention is between -1.47E-03 and -6.97E-04; the coefficient $A_6$ is between -7.56E-05 and -2.48E-05; the coefficient $A_8$ is between 2.13E-06 and 2.04E-05; and the coefficient $A_{10}$ is between -8.08E-07 and -2.42E-08.

As far as IOLs of the present invention formed of hydrophobic acrylate of a refractive index of 1.46 and having a diopter between 5D and 36D are concerned, in order to compensate a cornea spherical aberration between +0.28$\mu$m and +0.55$\mu$m, the coefficient $A_4$ in the aspherical surface curve expression of the IOLs of the present invention is between -4.528593E-03 and -7.675947E-04; the coefficient $A_6$ is between -1.634187E-04 and -2.068180E-05; the coefficient $A_8$ is between -3.422158E-05 and 1.528260E-05; and the coefficient $A_{10}$ is between -4.211297E-07 and 2.092757E-06.

As far as IOLs of the present invention formed of hydrophobic acrylate of a refractive index of 1.55 and having a diopter between 5D and 36D are concerned, in order to compensate a cornea spherical aberration between +0.28$\mu$m and +0.55$\mu$m, the coefficient $A_4$ in the aspherical surface curve expression of the IOLs of the present invention is between -9.621602E-04 and -4.490896E-04; the coefficient $A_6$ is between -6.053624E-05 and -1.437094E-05; the coefficient $A_8$ is between 5.293093E-07 and 8.700751E-06; and the coefficient $A_{10}$ is between -2.418869E-07 and 1.158933E-07.

To conclude, compared with the aspherical IOLs on the market, the new-type aspherical IOL of the present invention can correct a greater cornea spherical aberration so that the cataract patient enjoys better visual quality.
The embodiments described above are only exemplary and not restrictive. Therefore, without departing from the inventive concept disclosed in the description, those skilled in the art may modify or change the above embodiments. Hence, the protection scope of the present invention is only defined by the appended claims.

**Claims**

1.  An aspherical intraocular lens (IOLs), the surface shape of an anterior surface of an effective optical area of the aspherical intraocular lens being aspherical, or the surface shape of a posterior surface of an effective optical area of the aspherical intraocular lens being aspherical, or the surface shapes of an anterior surface and a posterior surface of an effective optical area of the aspherical intraocular lens being aspherical, **characterized in that** the spherical aberration of the aspherical intraocular lens is -0,28μm or -0,29μm.

2.  The aspherical IOL according to claim 1, **characterized in that** a curve of the aspherical surface shape on a two-dimensional coordinate system plane (YZ) satisfies the following aspherical surface curve expression:

$$Z(y) = \frac{cy^2}{1 + \sqrt{1 - c^2 y^2}} + \sum_{i=2}^{5} A_{2i} \bullet y^{2i}$$

   wherein Z(y) is an expression of the curve of the aspherical surface of the IOL effective optical area on the YZ plane, c is a reciprocal of a radius of curvature of the surface of the basic spherical surface of the effective optical area, y is a vertical distance of any point on the curve from the horizontal coordinate axis (Z), $A_{21}$ is a higher-order term coefficient of the aspherical surface,
   points on the aspherical surface shape are obtained in a way that the curve rotates about the horizontal coordinate axis (Z) for symmetry variation.

3.  The aspherical IOL (2) according to claim 2, **characterized in that** the aspherical IOL (2) is formed of silicone, hydrogel, hydrophobic acrylate, or polymethyl methacrylate (PMMA) of a refractive index ranging from 1.45 to 1.56 under the condition of 25°C.

4.  The aspherical IOL (2) according to claim 3, **characterized in that** the aspherical IOL (2) is formed of hydrophobic acrylate of a refractive index of 1.48 under the condition of 25°C, and that the diopter of the aspherical IOL is between 5D and 36D.

5.  The aspherical IOL (2) according to claim 4, **characterized in that** the coefficient $A_4$ in the aspherical surface curve expression is between -2.89E-03 and -7.00E-04; the coefficient $A_6$ is between -2.36E-04 and -4.50E-06; the coefficient $A_8$ is between -4.04E-05 and -4.23E-05; and the coefficient $A_{10}$ is between -1.87E-06 and -2.75E-06.

6.  The aspherical IOL (2) according to claim 1, **characterized in that** the coefficient $A_4$ in the aspherical surface curve expression is between -1.47E-03 and -6.91E-04; the coefficient $A_6$ is between -1.20E-04 and -3.56E-05; the coefficient $A_8$ is between 5.26E-06 and 1.37E-05; and the coefficient $A_{10}$ is between -3.71E-07 and -6.56E-08.

7.  The aspherical IOL (2) according to claim 1, **characterized in that** the coefficient $A_4$ in the aspherical surface curve expression is between -1.47E-03 and -6.97E-04; the coefficient $A_6$ is between -7.56E-05 and -2.48E-05; the coefficient $A_8$ is between 2.13E-06 and 2.04E-05; and the coefficient $A_{10}$ is between -8.08E-07 and -2.42E-08.

8.  The aspherical IOL (2) according to any one of the preceding claims, **characterized in that** a radius of curvature of a basic spherical surface of the effective optical area posterior surface of the aspherical IOL (2) is smaller than that of a basic spherical surface of the effective optical area anterior surface of the aspherical IOL (2).

9.  The aspherical IOL (2) according to any one of the preceding claims, **characterized in that** the aspherical IOL (2) is a one-piece IOL or a three-piece IOL.

**Patentansprüche**

1. Asphärische Intraokularlinse (IOLs), wobei die Oberflächenform einer vorderen Oberfläche eines wirksamen optischen Bereichs der asphärischen Intraokularlinse asphärisch ist, oder die Oberflächenform einer hinteren Oberfläche eines effektiven optischen Bereichs der asphärischen Intraokularlinse asphärisch ist, oder die Oberflächenformen einer vorderen Oberfläche und einer hinteren Oberfläche eines effektiven optischen Bereichs der asphärischen Intraokularlinse asphärisch sind, **dadurch gekennzeichnet, dass** die sphärische Aberration der asphärischen Intraokularlinse -0,28μm oder -0,29μm beträgt.

2. Asphärische IOL nach Anspruch 1, **dadurch gekennzeichnet, dass** eine Kurve der asphärischen Oberflächenform auf einer zweidimensionalen Koordinatensystem-Ebene (YZ) den folgenden Ausdruck der asphärischen Oberflächenkurve erfüllt:

$$Z(y) = \frac{cy^2}{1 + \sqrt{1 - c^2 y^2}} + \sum_{i=2}^{5} A_{2i} \bullet y^{2i}$$

wobei Z(y) ein Ausdruck der Kurve der asphärischen Oberfläche des IOL-effektiven optischen Bereichs auf der YZ-Ebene ist, c ein Kehrwert eines Krümmungsradius der Oberfläche der Oberfläche der sphärischen Grundfläche des effektiven optischen Bereichs ist, y ein vertikaler Abstand eines beliebigen Punktes der Kurve von der horizontalen Koordinatenachse (Z) ist, $A_{2i}$ ein Term-Koeffizient höherer Ordnung der asphärischen Oberfläche ist,
Punkte auf der asphärischen Oberflächenform so erhalten werden, dass sich die Kurve zur Symmetrievariation um die horizontale Koordinatenachse (Z) dreht.

3. Asphärische IOL (2) nach Anspruch 2, **dadurch gekennzeichnet, dass** die asphärische IOL (2) aus Silikon, Hidrogel, hydrophobem Acrylat oder Polymethylmethacrylat (PMMA) mit einem Brechungsindex von 1,45 bis 1,56 unter der Bedingung von 25°C gebildet ist.

4. Die asphärische IOL (2) nach Anspruch 3, **dadurch gekennzeichnet, dass** die asphärische IOL (2) aus hydrophobem Acrylat mit einem Brechungsindex von 1,48 unter der Bedingung von 25 °C gebildet ist und dass der Diopter der asphärischen IOL zwischen 5D und 36D liegt.

5. Asphärische IOL (2) nach Anspruch 4, **dadurch gekennzeichnet, dass** der Koeffizient $A_4$ im Ausdruck der asphärischen Oberflächenkurve zwischen -2,89E-03 und -7,00E-04 liegt; der Koeffizient $A_6$ zwischen -2,36E-04 und -4,50E-06 liegt; der Koeffizient $A_8$ zwischen -4,04E-05 und -4,23E-05 liegt; und der Koeffizient $A_{10}$ zwischen -1,87E-06 und -2,75E-06 liegt.

6. Asphärische IOL (2) nach Anspruch 1, **dadurch gekennzeichnet, dass** der Koeffizient $A_4$ im Ausdruck der asphärischen Oberflächenkurve zwischen -1,47E-03 und -6,91E-04 liegt; der Koeffizient $A_6$ zwischen -1,20E-04 und -3,56E-05 liegt; der Koeffizient $A_8$ zwischen -5,26E-06 und -1,37E-05 liegt; und der Koeffizient $A_{10}$ zwischen -3,71E-07 und -6,56E-08 liegt.

7. Asphärische IOL (2) nach Anspruch 1, **dadurch gekennzeichnet, dass** der Koeffizient $A_4$ im Ausdruck der asphärischen Oberflächenkurve zwischen -1,47E-03 und -6,97E-04 liegt; der Koeffizient $A_6$ zwischen -7,56E-05 und -2,48E-05 liegt; der Koeffizient $A_8$ zwischen -2,13E-06 und -2,04E-05 liegt; und der Koeffizient $A_{10}$ zwischen -8,08E-07 und -2,42E-08 liegt.

8. Die asphärische IOL (2) nach einem der vorhergehenden Ansprüche, **dadurch gekennzeichnet, dass** ein Krümmungsradius einer grundlegenden sphärischen Oberfläche des effektiven optischen Bereichs der hinteren Oberfläche der asphärischen IOL (2) kleiner ist als der einer grundlegenden sphärischen Oberfläche des effektiven optischen Bereichs der vorderen Oberfläche der asphärischen IOL (2).

9. Die asphärische IOL (2) nach einem der vorhergehenden Ansprüche, **dadurch gekennzeichnet, dass** die asphärische IOL (2) eine einteilige IOL oder eine dreiteilige IOL ist.

**Revendications**

1. Lentille intraoculaire (IOL) asphérique, la forme de surface d'une surface antérieure d'une zone optique efficace de la lentille intraoculaire asphérique étant asphérique, ou la forme de surface d'une surface postérieure d'une zone optique efficace de la lentille intraoculaire asphérique étant asphérique, ou les formes de surface d'une surface antérieure et d'une surface postérieure d'une zone optique efficace de la lentille intraoculaire asphérique étant asphérique, **caractérisée en ce que** l'aberration sphérique de la lentille intraoculaire asphérique est de -0,28 μm ou de -0,29 μm.

2. IOL asphérique selon la revendication 1, **caractérisée en ce qu'**une courbe de la forme de surface asphérique sur un plan (YZ) de système de coordonnées à deux dimensions satisfait l'expression de courbe de surface asphérique suivante :

$$Z(y) = \frac{cy^2}{1+\sqrt{1-c^2y^2}} + \sum_{i=2}^{5} A_{2i} \bullet y^{2i}$$

dans laquelle Z(y) représente une expression de la courbe de la surface asphérique de la zone optique efficace de l'IOL sur le plan (YZ), c représente le réciproque d'un rayon de courbure de la surface de la surface sphérique basique de la zone optique efficace, y représente une distance verticale d'un point quelconque situé sur la courbe à partir de l'axe de coordonnées horizontale (Z), A21 représente un coefficient de terme à ordre supérieur de la surface asphérique,
les points sur la forme de surface asphérique sont obtenus d'une manière telle que la courbe tourne autour de l'acte de coordonnées horizontale (Z) destiné à une variation de symétrie.

3. IOL asphérique (2) selon la revendication 2, **caractérisée en ce que** l'IOL asphérique(2) est formée de silicone, d'un hydrogène, d'acrylate hydrophobe ou de méthacrylate de polyméthyle (PMMA) ayant un indice de réfraction compris entre 1,45 et 1,56 sous la condition 25 °C.

4. IOL asphérique (2) selon la revendication 3, **caractérisée en ce que** l'IOL asphérique(2) est formée d'acrylate hydrophobe ayant un indice de réfraction de 1,48 sous la condition 25 °C, et **en ce que** le dioptre de l'IOL asphérique est compris entre 5 D et 36 D.

5. IOL asphérique (2) selon la revendication 4, **caractérisée en ce que** le coefficient A4 dans l'expression de courbe de surface asphérique est compris entre -2,89E-03 et -7,00E-04 ; le coefficient A6 est compris entre -2,36E-04 et -4,50E-06 ; le coefficient A8 est compris entre -4,04E-05 et -4,23E-05 ; et le coefficient A10 est compris entre -1,87E-06 et -2,75E-06.

6. IOL asphérique (2) selon la revendication 1, **caractérisée en ce que** le coefficient A4 dans l'expression de courbe de surface asphérique est compris entre -1,47E-03 et -6,91E-04 ; le coefficient A6 est compris entre -1,20E-04 et -3,56E-05 ; le coefficient A8 est compris entre -5,26E-06 et -1,37E-05 ; et le coefficient A10 est compris entre -3,71E-07 et -6,56E-08.

7. IOL asphérique (2) selon la revendication 1, **caractérisée en ce que** le coefficient A4 dans l'expression de courbe de surface asphérique est compris entre -1,47E-03 et -6,97E-04 ; le coefficient A6 est compris entre -7,56E-05 et -2,48E-05 ; le coefficient A8 est compris entre -2,13E-06 et -2,04E-05 ; et le coefficient A10 est compris entre -8,08E-07 et -2,42E-08.

8. IOL asphérique (2) selon l'une quelconque des revendications précédentes, **caractérisée en ce qu'**un rayon de courbure d'une surface sphérique basique de la surface postérieure de zone optique efficace de l'IOL asphérique(2) est inférieur à celui d'une surface sphérique basique de la surface antérieure de zone optique efficace de l'IOL asphérique(2).

9. IOL asphérique (2) selon l'une quelconque des revendications précédentes, **caractérisée en ce que** l'IOL asphérique(2) est une IOL à une pièce ou une IOL à trois pièces.

Fig. 1

Fig. 2

Fig. 3

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 20244628 U

**Non-patent literature cited in the description**

- **KOOI LING LIM ; BOPTOM (HONS ; HAN BOR FAM.** FRCSE. Ethnic differences in higher-order aberrations: Spherical aberration in the South East Asian Chinese eye. *J Cataract refractive surg,* 2009, vol. 35, 2144-2148

- **YANWEN FANG ; YI LU ; XINHUA WU ; AIZHU MIAO ; YI LUO.** Visual function and subjective quality of life in Chinese cataract patients after implantation with aspheric intraocular lenses. *Eur J Ophthalmol.,* 2011, vol. 21 (6), 732-740